# EUROPEAN PATENT SPECIFICATION

(11) **EP 1 454 652 B1**
(45) Date of publication and mention of the grant of the patent: **26.04.2006**
(21) Application number: 04425104.9
(22) Date of filing: 19.02.2004
(51) Int. Cl.: A61N 1/30

(54) **A device for transcutaneous administration of substances by means of iontophoresis**
Vorrichtung zur transdermalen Abgabe von Substanzen mittels Iontophorese
Dispositif pour l'administration transcutanée de substances en utilisant l'iontophorèse

(30) Priority: 07.03.2003 IT rn20030005
(43) Date of publication of application: 08.09.2004
(73) Proprietor: M & T S.r.l., 47900 Rimini (IT)
(72) Inventor: Tedoldi, Ezio, 42048 Rubiera (Reggio Emilia) (IT)
(74) Representative: Lanzoni, Luciano

(56) References cited:
- WO-A-00/10640
- WO-A-00/56400
- WO-A-02/09807
- GB-A- 2 372 705
- US-A- 5 983 131

## Description

The invention relates to a device for the transcutaneous administration of substances by means of iontophoresis.

Transcutaneous iontophoresis is a known technique for administering substances to the body through the skin in localised fashion and with minimum haematic absorption of the substances, reducing the risk of side effects linked to their uncontrolled diffusion. Some substances which can be administered may be, by way of example, drugs, herbal remedies, cosmetic and homeopathic products. Transcutaneous iontophoresis is used, for instance, in aesthetic medicine (for instance in the treatment of cutaneous tissue blemishes) or in pain relieving, relaxing or tonicising therapies.

Transcutaneous iontophoresis consists of letting an electrical current pass between a selected portion of skin and an electrode through a transport fluid interposed between the skin and the electrode and in which are dissolved or dispersed the molecules of the substance to be administered (usually in ionic, or electrolytic, or otherwise electrically charged form). The electrical circuit is closed applying one or more counter-electrodes on different parts of the skin from the treated one. The electrical current is applied according to an appropriate time-based wave form which allows to transfer the substances at a predetermined depth into the skin and based on predetermined mean square values over time.

The transport fluid is usually in the form of a gel.

Appropriate devices are constructed to apply this technique.

In particular, known devices for the transcutaneous administration of substances by means of iontophoresis comprise a hollow operative head made of plastic material, provided with two openings on two opposite sides. An opening, into which is inserted a container of the fluid, acts as an inlet for the fluid. The other opening is closed to measure, except for a minimum space along the long edges, by a plastic roller, free to rotate about its own axis and acting as a dispenser of the transport fluid. The part of the roller inside the cavity is in contact with the fluid coming from the container and it rotates, by effect of friction with the skin, when the roller is applied to the skin with the device kept in motion. The roller thereby brings the part covered with fluid in contact with the skin, distributing the fluid. To continue dispensing the fluid, the roller must thus be maintained in continuous rotation. A thin lamina folded in the shape of an "L" is inserted into the chamber as an electrode. The short side of the lamina is fastened to the wall of the operative head and it bears an electrical terminal external to the head, connected with a voltage driven current generator (which, in turn, is connected to one or more counter-electrodes). The long side of the lamina faces the roller along the axis of rotation with a plane of lay converging on the axis itself and practically nearly in contact therewith. The electrode thus applies voltage at a very short distance from the surface of the roller and the current, to reach the skin, must travel through the whole circumference of a section of the roller within the very thin layer of fluid adhering to its surface. Given the characteristics of the fluid, the electrical impedance of this segment is usually high. Furthermore, it is highly variable and its value cannot be reliably controlled. The thickness of the fluid layer distributed on the cylinder in the outer part is variable over time according to the conditions of the skin, of the pressure exerted thereon by the roller, of the deformation of the operative head due to the pressure of the operator's fingers, which alters the geometry of the long sides of the aperture whereto the roller is applied. Moreover, when the skin is already moistened by the fluid, friction on the skin may not be sufficient to cause the roller to rotate (unless a strong pressure is exerted on the skin, which can be annoying for the patient). The roller thus dries up, stopping its distribution of fluid, and the electrical impedance imposed on the generator increases. Additionally, the lamina, which is fastened only on one side and very thin, can tend to deform elastically. The device is voltage driven and it maintains a given current (according to a certain waveform). Therefore, impedance variations may give rise to voltage fluctuations and peaks which can cause discomfort and even small bums to the patient. Moreover, the plastic roller is easily scratched and deteriorates, diminishing its ability to rotate and drive the fluid and becoming difficult to clean. In addition, it cannot be easily sterilised.

Some examples of the above mentioned prior art are given in document WO0056400 which describes a device in which the dispensing element can also be a piece of felt or other permeable body. The dispensing element, when formed from electrically conducting material, is brought into electrical contact with the energy supply component, to apply an electrical potential directly to the epidermis.

Other kinds of iontophoresis equipments are described in literature. For example, WO 0209807 describes an equipment in which a flask, containing the molecules to be transported, is connected either with an elongated conductive element inserted into the flask or with a dispenser which could be for example a roller, an osmotic membrane, a pad of sponge or a perforated cap.

US 5983131 discloses the preamble of claim 1 and describes an apparatus which includes a perforate electrically insulating layer, which presents on one side of it a first electrode which is proximate to the target tissue of the subject's body surface and on the other side a second electrode. An electric field extending between said electrodes will preferentially extend through perforations of the electrically insulating layer. Either the electrodes or the electrically insulating layer are placed into a reservoir containing the active principle and the fluid carrier. The electric current generated for the treatment penetrates only into the surface layer of the skin and then comes back to the dispensing element, to close the circuit.

WO0010640 describes an apparatus which provides an improved method for the administration of a useful substance into and across a body surface.

Either the useful substance is placed in capillary tubes which prevent dripping, or a filler of absorbent material is soaked with said useful substance and placed into contact with emitter rods, thus again preventing dripping. Ionized molecular particles are emitted from capillary tubes or rods and accelerated in the form of a particle beam to bombard from a distance the body surface under the influence of a strong electric field. In the course of operation no fluid is in contact with the surface of the body of the patient, but only a beam of charged molecular particles flies from the emitter to the skin. The process is fundamentally distinguished from conventional, electrically driven transdermal drug administration methods such as iontophoresis.

The objects achieved by the present invention are to enable easy adaptation of the device to all requirements or skin areas and easy cleaning/sterilising of the operative head.

This is achieved, in accordance with the present invention, by a device for the transcutaneous administration of substances by means of iontophoresis having structural and functional characteristics in accordance with the appended independent claim, additional embodiments of said device being identified in the appended dependent claims.

The invention is described in greater detail hereafter with the aid of the drawings, which represent an embodiment provided purely by way of non limiting example.
- Figure1 shows a partially sectioned side view of a device according to the invention with two details of as many variants of the dispenser (partially sectioned along the trace A-A).
- Figure 2 shows a schematic perspective view of an embodiment of the device with detail of the operative head.

In the present context and unless otherwise expressly stated, the term "cylinder" generally means the solid enclosed by the straight lines (called "generatrices") conducted mutually parallel by the points of a closed line (called "directrix") whose shape may also be different from circular and, at the limit, even polygonal (in which case the "cylinder" would be identified with a prism). If the bases or ends of the cylinder are perpendicular to the generatrices, the cylinder is called "straight".

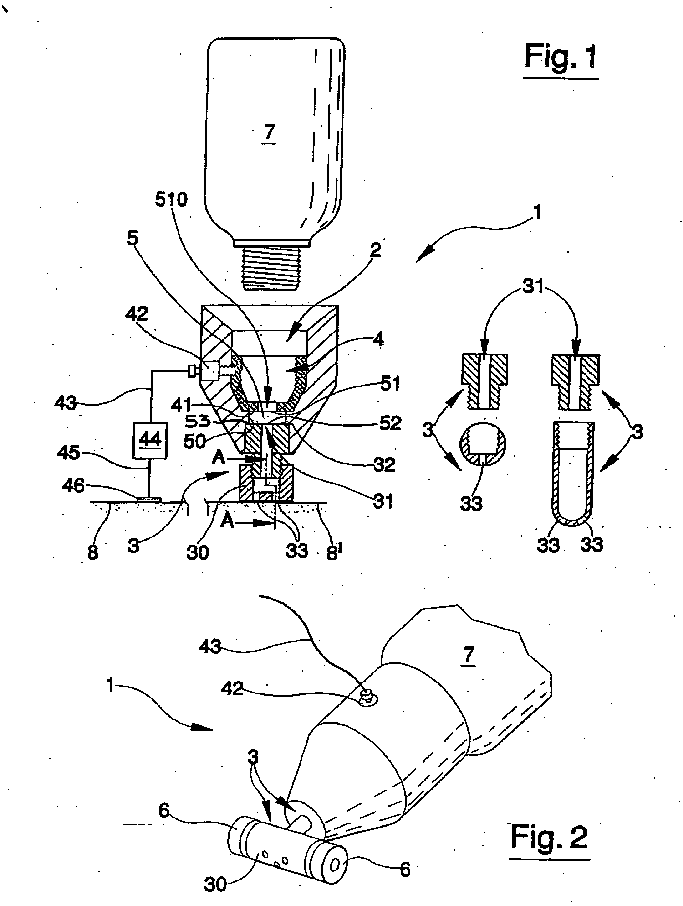

With reference to the figures, a device for the transcutaneous administration of substances by means of iontophoresis comprises an operative head 1 provided with an inlet 2 for a fluid transporting substances to be administered, with a fluid dispenser 3 which has a part 30 in contact with the skin, with an electrode 4 positioned on a path of the fluid from the inlet 2 to the dispenser 3. In general, the inlet 2 can be connected in various manners to a source or tank of fluid and the operative head 1 can be directly hand held to operate on the patient. Conveniently, as in prior art solutions, the device may comprise a container 7 of the fluid coupled with the inlet 2 in various manners. It may be fixed (and, in this case, the container 7 must be refillable or, at least, be a housing of a replaceable fluid cartridge) or removable (for example, as in Figure 1, by means of a threaded coupling).

Generally, the operative head 1 has a main body made of insulating material which, as shown in the figures, may have a first straight cylindrical part with circular cross section and a second cone frustum shaped or slightly tapered part to facilitate its handling. In regards to the characteristics of the transport fluid and of the substances to be administered, what has been stated above applies. The fluid is preferably a gel. The substances can also be dissolved into it in electrolytic form.

Obviously, as provided by iontophoresis technique, the electrode 4 is generally in contact with the external terminal 42, connected with a voltage driven current generator 44 by means of a first conductor cable 43. The generator 44 is in turn connected, by means of at least a second conductor cable 45, to at least a counter electrode 46 positioned on a first portion of skin 8, to close the circuit.

In the device according to the invention, the dispenser 3 is made of electrically conducting material and is in electrical contact with the electrode 4 only by the action of the fluid. Moreover, in combination, the dispenser 3 and the electrode 4 are arranged in a predetermined relative position which maintains constant over time the geometric shape of the volume of fluid present between them.

Therefore, the potential of a second portion of skin 8', whereon the contact part 30 acts, is carried by the metallic body of the dispenser 3 (minus the minimal voltage drop which takes place in the very thin layer of fluid on the skin) to the border of the geometric shape of the volume of fluid present between the dispenser 3 and the electrode 4, which remains constant over time. Only in said volume of fluid does current flow from the electrode 4 to the dispenser 3 and hence the difference in potential between the electrode 4 and the second portion of skin 8' is substantially given by the path of the current in said volume and, since the fluid is homogeneous and isotropic, the impedance seen by the generator always remains substantially constant once the geometry of the volume is defined. Obviously, the material whereof the body of the operative head 1 is made must be rigid or the thickness of the walls of the body of the operative head 1 must be sufficient to inhibit their deformation. A similar condition applies to the electrode 4 and to the dispenser 3. The dispenser 3, is rigid and made of a material which can also be a metal.

The dispenser 3 is fixed relative to the operative head 1, it has at least an inlet 31 for the inflow of the fluid into it and on its part 30 in contact with the skin it has at least a hole 33 for the outflow of the fluid towards the skin connected with the inlet 31. In this way, even if, for various reasons, no fluid should be present on the surface of the contact part 30, the hole 33 would always bring a part of the fluid (contained in the operative head 1) in contact with the skin, thus maintaining substantially unaltered the impedance characteristics seen by the generator 44. Advantageously, the dispenser 3 is made of stainless steel. In addition to being a good conductor of electricity, it can thus be easily cleaned and sterilised and does not deteriorate easily.

Conveniently, in the operative head 1 is obtained along the path of the fluid a segment 5 of predetermined length, which separates the electrode 4 from the dispenser 3, has electrically insulating lateral walls 50, has a first end 51 defined by a surface portion 41 of the electrode 4 and having at least a passage 510 for the fluid, has a second end 52 defined by a surface portion 32 of the dispenser 3. The geometry of the volume of fluid present between the electrode 4 and the device 3 is thereby defined in an exact and simple manner. In this case, when the dispenser 3 is fixed relative to the operative head 1 and has at least the inlet 31 for the entrance of the fluid in its interior and, on its part 30 in contact with the skin, it has at least the hole 33 for the outflow of the fluid towards the skin connected with the inlet 31, the inlet 31 is obtained in the second end 52 of the segment 5 of the path of the fluid.

Preferably, the surface portion 41 of the electrode 4 and the surface portion 32 of the dispenser 3 are plane.

Advantageously, moreover, as shown in Figure 1, the segment 5 is straight cylindrical with predetermined section and the first and the second end 51, 52 are its bases. Said ends may be circular. The geometry of the segment 5 is thus particularly simple.

In general, advantageously the device may comprise means (not shown herein) for regulating the relative position of the dispenser 3 and of the electrode 4, to vary or control in predetermined fashion the impedance imposed on the generator 44 according to the type of fluid or of electrolyte and/or of substance to be administered, or according to the type of treatment and/or of skin. In particular, the device comprises means (not shown herein) for adjusting the distance between the planar surface portion 32 of the dispenser 3 and the planar surface portion 41 of the electrode 4. Said means can be of various kinds.

Preferably, they may provide for adjustment with micrometre screw acting either on the dispenser 3 or on the electrode 4 (or on parts thereof) in such a way as to make them slide in guided fashion along the axis of the segment 5. In a preferred embodiment of the invention shown in Figure 1, the dispenser 3 is at least partially inserted in a cylindrical conduit whose lateral surface defines the lateral walls 50 of the straight cylindrical segment 5 of the fluid path. The position adjustment means can thus be applied in very simple fashion, using the cylindrical conduit directly as a guide (in this case, the cylindrical conduit being straight and, preferably, with circular cross section).

The definition of the value of the surface area of the section of the cylindrical segment 5 can also be used to set the impedance value.

Advantageously, along the cylindrical conduit is obtained a small abutment 53 for arresting the insertion of the dispenser 3 into the conduit, which defines a minimum value of the length of the straight cylindrical segment 5. By inserting diaphragms it is possible to modify the position of the abutment and, hence, the minimum length of the segment 5 (in the absence of micrometre adjustments, this can also be a coarse system for varying or controlling impedance).

Moreover, advantageously, as shown in Figure 1, in the part 30 of the dispenser 3 in contact with the skin are obtained at least two holes 33 in predetermined position. In the part 30 of the dispenser 3 in contact with the skin can also be obtained a plurality of holes 33 distributed according to a predetermined pattern. With reference to figure 1 where three examples of dispenser 3 are shown (one is shown in the operative head 1 and two are shown separately), according to the invention the part 30 adapted to be in contact with the skin is removable. This allows either to clean it and/or sterilise it separately or to replace it with another one, contoured and shaped differently for a different use. In particular, in addition to a simple cylindrical shape (as shown in Figure 2, and possibly with ample axial dimension in order simultaneously to treat an ample area of skin), the part 30 in contact with the skin can have a special contour which allows it to reach small or hidden parts of skin. In particular, the part 30 in contact with the skin is elongated and has reduced cross section (for instance in the form of a long "nose"). The association between the part 30 in contact with the skin and the remaining part of the dispenser 3 can, for instance, be achieved with a threaded coupling (as shown in Figure 1) or with another type of coupling. As also shown in Figure 1, the portion 30 in contact with the skin can be hollow or, otherwise, comprise a compensation compartment downstream of a segment of connecting conduit between the inlet 31 and the contact part 30 itself, into which is collected a certain quantity of fluid which can assure a continuous and constant dispensing of fluid from the hole or holes 33. As shown in Figure 2, the device can comprise rolling friction means 6 to facilitate the motion on the skin of the part 30 in contact with the skin. However, this additional characteristic is not necessary: if the steel whereof the portion 30 in contact with the skin is made is well polished, the presence of even a very small quantity of fluid is sufficient to assure its ability to slide.

In the particular embodiment specifically illustrated in Figure 1, the electrode 4 is in the form of a bushing (for instance also made of stainless steel), inserted coaxially to the body of the operative head 1 in a related seat and frontally bearing a planar part which constitutes the surface portion 41 of the electrode 4. The interior of the bushing can easily be threaded. This form of electrode, particularly simple, rigid and solid, is particularly suitable for the invention.

As is readily apparent from the description, the invention achieves important advantages.

First of all, problems to the patient, due to impedance variability, are prevented.

Secondly, load impedance can be adjusted accurately according to the characteristics of the fluid, to the type of treatment or of skin.

An additional, important advantage is that the device can easily be adapted to all requirements or skin areas.

Moreover, a no less important advantage consists of the ability to clean and sterilise the operative head with ease, or just the part 30 of the dispenser 3 in contact with the skin.

## Claims

1. Device for the transcutaneous administration of substances by means of iontophoresis, comprising an operative head (1) provided with an inlet (2) for a fluid transporting substances to be administered, with a fluid dispenser (3) which has a part (30) adapted to be in contact with the skin, with an electrode (4) positioned on a path of the fluid from the inlet (2) to the dispenser (3), wherein the dispenser (3) is made of electrically conducting material and is in electrical contact with the electrode (4) only by the action of the fluid; the dispenser (3) and the electrode (4) are arranged in a predetermined relative position which maintains constant over time the geometric shape of the volume of fluid present between them; the dispenser (3) is fixed relative to the operative head (1), has at least an inlet (31) for the entrance of the fluid in its interior and on its part (30) adapted to be in contact with the skin it has at least a hole (33) for the outflow of the fluid towards the skin, connected with the inlet (31); the electrode (4) is generally in contact with an external terminal (42), connected with a voltage driven current generator (44) by means of a first conductor cable (43); the generator (44) in turn, is connected, by means of a second conductor cable (45), to at least a counter-electrode (46) adapted to be positioned on a first portion of skin (8) to close the circuit, the part (30) adapted to be in contact with the skin is adapted to be acting on a second portion of skin (8'), **characterised in that** the part (30) adapted to be in contact with the skin is removable from the dispenser (3).

2. A device as claimed in claim 1, **characterised in that** it comprises means for adjusting the relative position of the dispenser (3) and of the electrode (4).

3. A device as claimed in claim 1 or 2, **characterised in that** in the operative head (1) is obtained along the path of the fluid a segment (5) with predetermined length, which separates the electrode (4) from the dispenser (3), has electrically insulating lateral walls (50), has a first end (51) defined by a surface portion (41) of the electrode (4) and having at least a passage (510) for the fluid, has a second end (52) defined by a surface portion (32) of the dispenser (3).

4. A device as claimed in claim 3, **characterised in that** the surface portion (41) of the electrode (4) and the surface portion (32) of the dispenser (3) are planar.

5. A device as claimed in claim 4, **characterised in that** the segment (5) is straight cylindrical with predetermined cross section and the first and the second end (51,52) are its bases.

6. A device as claimed in claim 5, **characterised in that** the dispenser (3) is at least partially inserted in a cylindrical conduit whose lateral surface defines the lateral walls (50) of the straight cylindrical segment (5) of the path of the fluid.

7. A device as claimed in claim 6, **characterised in that** along the conduit is obtained an abutment (53) for arresting the insertion of the dispenser (3) into the conduit, which defines a minimum value of the length of the straight cylindrical segment (5).

8. A device as claimed in any of the claims from 3 through 7, **characterised in that** it comprises means for adjusting the distance between the planar surface portion (32) of the dispenser (3) and the planar surface portion (41) of the electrode (4).

9. A device as claimed in any of the claims from 3 through 8, **characterised in that** the inlet (31) is obtained in the second end (52) of the segment (5) of the path of the fluid.

10. A device as claimed in any of previous claims, **characterised in that** in the part (30) of the dispenser (3) adapted to be in contact with the skin is obtained a plurality of holes (33) distributed according to a predetermined pattern.

11. A device as claimed in any of previous claims, **characterised in that** the part (30) adapted to be in contact with the skin is elongated and has reduced cross section, in such a way as to reach small or hidden parts of skin.

12. A device as claimed in any of previous claims, **characterised in that** it comprises rolling friction means (6) to facilitate the motion on the skin of the part (30) adapted to be in contact with the skin.

13. A device as claimed in any of the previous claims, **characterised in that** the dispenser (3) is made of stainless steel.

14. A device as claimed in any of the previous claims, **characterised in that** it comprises a container (7) of the fluid coupled to the inlet (2).

## Patentansprüche

1. Vorrichtung zur transdermalen Abgabe von Substanzen mittels Iontophorese, enthaltend einen operativen Kopf (1), der mit einem Einlass (2) für ein die zu verabreichenden Substanzen transportierendes Fluid versehen i st, s o-wie mit einem Fluidspender (3), welcher ein für den Kontakt mit der Haut bestimmtes Teil (30) hat, und mit einer Elektrode (4), angeordnet an einer Bahn des Fluids von dem Einlass (2) bis zu dem Spender (3), wobei der Spender (3) aus elektrisch leitendem Material hergestellt ist und sich mit der Elektrode (4) nur durch die Wirkung des Fluids in elektrischem Kontakt befindet; wobei der Spender (3) und die Elektrode (4) in einer vorgegebenen entsprechenden Position angeordnet sind, welche auf Dauer die geometrische Form des Fluidvolumens konstant hält, das zwischen diesen vorhanden ist; wobei der Spender (3) im Verhältnis zu dem operativen Kopf (1) feststehend ist und wenigstens einen Einlass (31) für den Eintritt des Fluids in sein Inneres aufweist, und an seinem für den Kontakt mit der Haut bestimmten Teil (30) wenigstens eine Bohrung (33) für das Ausfliessen des Fluids in Richtung der Haut hat, die an den Einlass (31) angeschlossen ist; wobei sich die Elektrode (4) generell im Kontakt mit einem äusseren Terminal (42) befindet, verbunden mit einem spannungsgetriebenen Stromerzeuger (44) mit Hilfe eines ersten Leitungskabels (43); wobei der Stromerzeuger (44) wiederum mit Hilfe eines zweiten Leitungskabels (45) an wenigstens eine Zähler-Elektrode (46) angeschlossen ist, vorgesehen, auf einem ersten Hautabschnitt (8) positioniert zu werden, um den Kreis zu schliessen, wobei das für den Kontakt mit der Haut vorgesehene Teil (30) dazu bestimmt ist, auf einen zweiten Hautabschnitt (8') zu wirken, **dadurch gekennzeichnet, dass** das für den Kontakt mit der Haut vorgesehene Teil (30) von dem Spender (3) abnehmbar ist.

2. Vorrichtung nach Patentanspruch 1, **dadurch gekennzeichnet, dass** sie Mittel zum Einstellen der entsprechenden Position des Spenders (3) und der Elektrode (4) enthält.

3. Vorrichtung nach Patentanspruch 1 oder 2, **dadurch gekennzeichnet, dass** in dem operativen Kopf (1) entlang der Bahn des Fluids ein Segment (5) von einer vorgegebenen Länge erhalten ist, welches die Elektrode (4) von dem Spender (3) trennt, das elektrisch isolierende Seitenwände (50) hat, das ein erstes Ende (51) hat, welches durch einen Oberflächenabschnitt (41) der Elektrode (4) beschrieben ist und wenigstens einen Durchlass (510), für das Fluid aufweist, sowie ein zweites Ende (52), beschrieben durch einen Oberflächenabschnitt (32) des Spenders (3).

4. Vorrichtung nach Patentanspruch 3, **dadurch gekennzeichnet, dass** der Oberflächenabschnitt (41) der Elektrode (4) und der Oberflächenabschnitt (32) des Spenders (3) eben sind.

5. Vorrichtung nach Patentanspruch 4, **dadurch gekennzeichnet, dass** das Segment (5) gerade zylindrisch und von einem vorgegebenen Querschnitt ist, mit den ersten und zweiten Enden (51, 52) jeweils als seine Basis.

6. Vorrichtung nach Patentanspruch 5, **dadurch gekennzeichnet, dass** der Spender (3) wenigstens teilweise in eine zylindrische Leitung eingesetzt ist, deren seitliche Oberfläche die Seitenwände (50) des geraden zylindrischen Segmentes (5) der Bahn für das Fluid beschreibt.

7. Vorrichtung nach Patentanspruch 6, **dadurch gekennzeichnet, dass** entlang der Leitung ein Anschlag (53) zum Anhalten des Einsetzens des Spenders (3) in die Leitung erhalten ist, welcher einen Mindestwert der Länge des geraden zylindrischen Segmentes (5) festlegt.

8. Vorrichtung nach einem beliebigen der Patentansprüche von 3 bis 7, **dadurch gekennzeichnet, dass** sie Mittel zum Regulieren des Abstandes zwischen dem ebenen Oberflächenabschnitt (32) des Spenders (3) und dem ebenen Oberflächenabschnitt (41) der Elektrode (4) enthält.

9. Vorrichtung nach einem beliebigen der Patentansprüche von 3 bis 8, **dadurch gekennzeichnet, dass** der Einlass (31) in dem zweiten Ende (52) des Segmentes (5) der Bahn des Fluids erhalten ist.

10. Vorrichtung nach einem beliebigen der vorstehenden Patentansprüche, **dadurch gekennzeichnet, dass** in dem für den Kontakt mit der Haut vorgesehenen Teil (30) des Spenders (3) eine Anzahl von Bohrungen (33) erhalten sind, verteilt nach einem bestimmten Muster.

11. Vorrichtung nach einem beliebigen der vorstehenden Patentansprüche, **dadurch gekennzeichnet, dass** das für den Kontakt mit der Haut vorgesehene Teil (30) länglich ist und einen reduzierten Querschnitt hat, und zwar auf solche Weise, dass kleine oder versteckte Teile der Haut erreicht werden können.

12. Vorrichtung nach einem beliebigen der vorstehenden Patentansprüche, **dadurch gekennzeichnet, dass** sie Rollreibungsmittel (6) enthält, um die Bewegung des für den Kontakt mit der Haut vorgesehenen Teils (30) auf der Haut zu erleichtern.

13. Vorrichtung nach einem beliebigen der vorstehenden Patentansprüche, **dadurch gekennzeichnet, dass** der Spender (3) aus rostfreiem Stahl hergestellt ist.

14. Vorrichtung nach einem beliebigen der vorstehenden Patentansprüche, **dadurch gekennzeichnet, dass** sie einen Behälter (7) für das Fluid enthält, der an den Einlass (2) angeschlossen ist.

## Revendications

1. Dispositif pour l'administration transcutanée de substances en utilisant l'iontophorèse, comprenant une tête opératrice (1) pourvue d'une entrée (2) pour un fluide transportant des substances à administrer, avec un distributeur de fluide (3) présentant une partie (30) adaptée pour être en contact avec la peau, avec une électrode (4) positionnée sur un parcours du fluide de l'entrée (2) au distributeur (3), dans lequel le distributeur (3) est réalisé en un matériau électriquement conducteur et est en contact électrique avec l'électrode (4) par la seule action du fluide; le distributeur (3) et l'électrode (4) sont disposés dans une position relative prédéterminée qui maintient constante dans le temps la forme géométrique du volume de fluide présent entre eux; le distributeur (3) est fixe par rapport à la tête opératrice (1), présente au moins une entrée (31) pour l'entrée du fluide dans son intérieur, et sur sa partie (30) adaptée pour être en contact avec la peau il présente au moins un orifice (33) pour la sortie du fluide vers la peau, relié à l'entrée (31); l'électrode (4) est généralement en contact avec un terminal externe (42), connecté à un générateur de courant (44) actionné par la tension au moyen d'un premier cable conducteur (43); le générateur (44), à son tour, est connecté, au moyen d'un second cable conducteur (45), à au moins une contre-électrode (46) adaptée à être positionnée sur une première portion de peau (8) pour fermer le circuit, la partie (30) adaptée pour être en contact avec la peau étant adaptée pour agir sur une seconde portion de peau (8'), **caractérisé en ce que** la partie (30) adaptée pour être en contact avec la peau est amovible du distributeur (3).

2. Dispositif selon la revendication 1, **caractérisé en ce qu**'il comprend des moyens d'ajustement de la position relative du distributeur (3) et de l'électrode (4).

3. Dispositif selon la revendication 1 ou 2, **caractérisé en ce que** dans la tête opératrice (1) est obtenu le long du parcours du fluide un segment (5) avec une longueur prédéterminée, qui sépare l'électrode (4) du distributeur (3), a des parois latérales (50) isolantes électriquement, a une première extrémité (51) définie par une portion de surface (41) de l'électrode (4) et ayant au moins un passage (510) pour le fluide, a une seconde extrémité (52) définie par une portion de surface (32) du distributeur (3).

4. Dispositif selon la revendication 3, **caractérisé en ce que** la portion de surface (41) de l'électrode (4) et la portion de surface (32) du distributeur (3) sont planes.

5. Dispositif selon la revendication 4, **caractérisé en ce que** le segment (5) est cylindrique rectiligne avec une section transversale prédéterminée et que les première et seconde extrémités (51, 52) sont ses bases.

6. Dispositif selon la revendication 5, **caractérisé en ce que** le distributeur (3) est au moins partiellement inséré dans un conduit cylindrique dont la surface latérale définit les parois latérales (50) du segment cylindrique rectiligne (5) du parcours du fluide.

7. Dispositif selon la revendication 6, **caractérisé en ce que** le long du conduit est obtenue une butée (53) d'arrêt de l'insertion du distributeur (3) dans le conduit, laquelle butée définit une valeur minimum de la longueur du segment cylindrique rectiligne (5).

8. Dispositif selon n'importe laquelle des revendications 3 à 7, **caractérisé en ce qu'**il comprend des moyens d'ajustement de la distance entre la portion de surface plane (32) du distributeur (3) et la portion de surface plane (41) de l'électrode (4).

9. Dispositif selon n'importe laquelle des revendications 3 à 8, **caractérisé en ce que** l'entrée (31) est obtenue dans la seconde extrémité (52) du segment (5) du parcours du fluide.

10. Dispositif selon n'importe laquelle des revendications précédentes, **caractérisé en ce que** dans la partie (30) du distributeur (3) adapté pour être en contact avec la peau est obtenue une pluralité d'orifices (33) distribués selon un dessin prédéterminé.

11. Dispositif selon n'importe laquelle des revendications précédentes, **caractérisé en ce que** la partie (30) adaptée pour être en contact avec la peau est allongée et présente une section transversale réduite, de manière à atteindre des parties réduites ou cachées de peau.

12. Dispositif selon n'importe laquelle des revendications précédentes, **caractérisé en ce qu'**il comprend des moyens de friction roulants (6) pour faciliter le mouvement sur la peau de la partie (30) adaptée pour être en contact avec la peau.

13. Dispositif selon n'importe laquelle des revendications précédentes, **caractérisé en ce que** le distributeur (3) est réalisé en acier inoxydable.

14. Dispositif selon n'importe laquelle des revendications précédentes, **caractérisé en ce qu'**il comprend un conteneur (7) de fluide accouplé avec l'entrée (2).
